# EUROPEAN PATENT APPLICATION

(11) **EP 3 512 301 A1**
(43) Date of publication of application: **17.07.2019**
(21) Application number: 18173383.3
(22) Date of filing: 19.05.2018
(51) Int. Cl.: H05B 3/42, A24F 47/00

(54) **ROUND ROD-SHAPED CERAMIC HEATING ELEMENT FOR ELECTRONIC CIGARETTE**

(30) Priority: 16.01.2018 CN 201820069756 U
(71) Applicant: Key Material Co., Ltd., 523560 Dongguan City GuangDong Province (CN)
(72) Inventor: Huang, Tao, Wuhan City, Hubei Province (CN); Chen, Zhijian, Yingde City, Guangdong (CN)
(74) Representative: Cabinet Chaillot

(57) **Abstract**

The invention discloses a round rod-shaped ceramic heating element for an electronic cigarette, which comprises a ceramic core rod, a No. 1 ceramic substrate, a heating circuit, a No. 2 ceramic substrate, a temperature measuring circuit and a plurality of bonding pads with metal leads, wherein a ceramic flange is sleeved on an outer circumferential surface of the ceramic core rod. The invention has the following beneficial effects: the heating speed is fast, and the temperature in a heating zone can reach above 300°C in 12 seconds; a temperature measuring resistor responds quickly and the temperature is accurately controlled; and its structure is stable and unlikely to be broken.

## Description

### FIELD OF THE INVENTION

The invention relates to the application field of electronic cigarettes, and in particular to a round rod-shaped ceramic heating element for an electronic cigarette.

### BACKGROUND OF THE INVENTION

In recent years, novel flue-cured tobaccos have been introduced to the market such that traditional electronic cigarette heaters have failed to meet their requirements on heating speed, heating uniformity and temperature control accuracy. Traditional heating wires have a small heating area, provide non-uniform heating and cannot control temperature accurately. Traditional barrel-shaped ceramic heating elements have a slow heating speed and severe heat loss.

In view of the above problems, the invention relates to a round rod-shaped ceramic heating element, which has the advantages of a small size, a high heating speed, accurate temperature control, and high heat utilization efficiency and little heat loss by being inserted directly into cigarettes to bake tobacco.

### SUMMARY OF THE INVENTION

An object of the invention is to provide a round rod-shaped ceramic heating element for an electronic cigarette so as to solve the above problems.

To achieve the above object, the invention employs the following technical solution: a round rod-shaped ceramic heating element for an electronic cigarette comprises a ceramic core rod, wherein a No. 1 ceramic substrate is arranged at an outermost side of the ceramic core rod, a heating circuit is arranged at an inner side of the No. 1 ceramic substrate, a No. 2 ceramic substrate is arranged at an inner side of the heating circuit, a temperature measuring circuit is arranged between the No. 2 ceramic substrate and the ceramic core rod, a plurality of bonding pads are arranged at an upper end of an outer circumferential surface of the No. 1 substrate, leads are arranged above the bonding pads, a ceramic flange is sleeved on an outer circumferential surface of the ceramic core rod, and each of the bonding pads is provided with a metal lead.

A heating resistor may be arranged at an outer side of the ceramic core rod and the inner side of the No. 1 substrate, and a temperature measuring resistor may be arranged beside the heating resistor.

The ceramic core rod may have a round rod-shaped tip structure with a tip angle of 30-140°.

The metal lead may be a nickel wire or a copper wire.

The ceramic core rod may be a solid structure or a partially hollow structure with blind holes at one end.

The ceramic core rod may be a ceramic core rod made of alumina having a purity of 75%-99%.

A protective coating may be arranged on the outer circumferential surface of the ceramic core rod, and the protective coating may be an oleophobic glass glaze. The round rod-shaped ceramic heating element for an electronic cigarette manufactured by the technical solution of the invention has the following beneficial effects: the heating speed is fast, and the temperature in a heating zone can reach above 300°C in 12 seconds; a temperature measuring resistor responds quickly and the temperature is accurately controlled; and its structure is stable and unlikely to be broken.

### BRIEF DESCRIPTION OF THE DRAWINGS

Fig. 1 is a schematic structural view of a round rod-shaped ceramic heating element for an electronic cigarette according to the invention; and
Fig. 2 is a front view of the round rod-shaped ceramic heating element for an electronic cigarette according to the invention.

In the Figures: 1. ceramic core rod; 2. heating circuit; 3. temperature measuring circuit; 4. No. 1 ceramic substrate; 5. bonding pad; 6. protective coating; 7. lead; 8. ceramic flange; 9. temperature measuring resistor; 10. heating resistor; 11. No. 2 ceramic substrate; 12. metal lead.

### DETAILED DESCRIPTION OF THE INVENTION

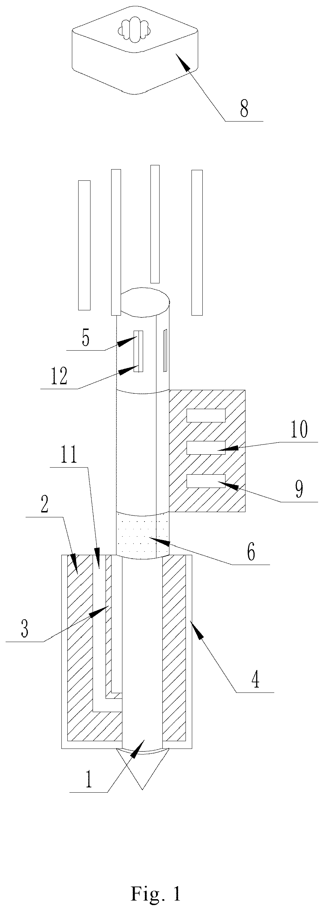

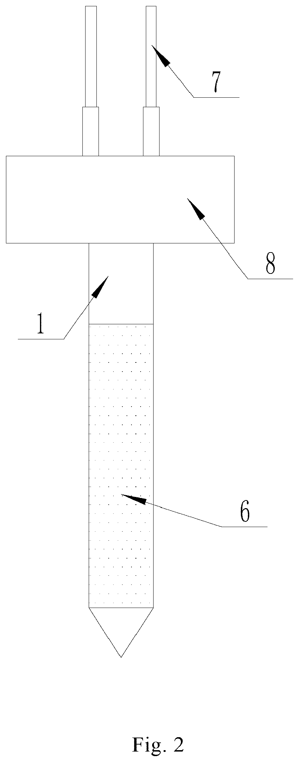

The invention will be specifically described below with reference to the drawings. As shown in Figs. 1 to 2, a round rod-shaped ceramic heating element for an electronic cigarette comprises a ceramic core rod 1, wherein a No. 1 ceramic substrate 4 is arranged at an outermost side of the ceramic core rod 1, a heating circuit 2 is arranged at an inner side of the No. 1 ceramic substrate 4, a No. 2 ceramic substrate 11 is arranged at an inner side of the heating circuit 2, a temperature measuring circuit 3 is arranged between the No. 2 ceramic substrate 11 and the ceramic core rod 1, a plurality of bonding pads 5 are arranged at an upper end of an outer circumferential surface of the No. 1 substrate 4, leads 7 are arranged above the bonding pads 5, a ceramic flange 8 is sleeved on an outer circumferential surface of the ceramic core rod 1, and each of the bonding pads 5 is provided with a metal lead 12; a heating resistor 10 is arranged at an outer side of the ceramic core rod 1 and the inner side of the No. 1 substrate 4, and a temperature measuring resistor 9 is arranged beside the heating resistor 10; the ceramic core rod 1 has a round rod-shaped tip structure with a tip angle of 30-140°; the metal lead 12 is a nickel wire or a copper wire; the ceramic core rod 1 has a solid structure or a partially hollow structure with blind holes at one end; the ceramic core rod 1 is a ceramic core rod made of alumina having a purity of 75%-99%; and a protective coating 6 is arranged on the outer circumferential surface of the ceramic core rod 1, and the protective coating 6 is an oleophobic glass glaze.

This embodiment is characterized in that: a No. 1 ceramic substrate is arranged at an outermost side of the ceramic core rod, a heating circuit is arranged at an inner side of the No. 1 ceramic substrate, a No. 2 ceramic substrate is arranged at an inner side of the heating circuit, a temperature measuring circuit is arranged between the No. 2 ceramic substrate and the ceramic core rod, a plurality of bonding pads are arranged at an upper end of an outer circumferential surface of the No. 1 substrate, leads are arranged above the bonding pads, a ceramic flange is sleeved on an outer circumferential surface of the ceramic core rod, and each of the bonding pads is provided with a metal lead. The invention has the following beneficial effects: the heating speed is fast, and the temperature in a heating zone can reach above 300°C in 12 seconds; a temperature measuring resistor responds quickly and the temperature is accurately controlled; and its structure is stable and unlikely to be broken.

In this embodiment, a heating circuit 2 and a temperature measuring circuit 3 are pre-printed on surfaces of two ceramic substrates (a No. 1 ceramic substrate 4 and a No. 2 ceramic substrate 11) coated onto the ceramic core rod 1; the heating circuit 2 and the temperature measuring circuit 3 are located between the two ceramic substrates and the ceramic core rod and isolated from the environment; a heating resistor 10 is arranged between the ceramic core rod 1 and the No. 1 ceramic substrate 4, a temperature measuring resistor 9 is arranged beside the heating resistor 10, and measurement is regulated and the temperature of the heating element is regulated according to a change in the resistance value of the temperature measuring circuit 3 or the temperature measuring resistor 9; three or four bonding pads 5 are arranged on an upper outer surface of the ceramic core rod 1 (i.e. the bonding pads 5 are printed on the other surface of the No. 1 ceramic substrate 4), and the bonding pads 5 are pre-punched, and communicated with electrodes by through holes filled with a metal slurry; among the three or four bonding pads, two bonding pads are provided with extraction electrodes of the heating circuit 2, the other one or two bonding pads are provided with extraction electrodes of the temperature measuring circuit 3, and the extraction electrode of one bonding pad is shared; each of the bonding pads 5 is respectively welded with one separate metal lead 12 by a high-temperature brazing process in which silver or a silver-copper alloy is used as a high-temperature brazing filler; leads 7 are arranged above the bonding pads 5, a ceramic flange 8 is sleeved on an outer circumferential surface of the ceramic core rod 1, and the ceramic core rod 1 has a round rod-shaped tip structure with a tip angle of 30-140°; the metal lead 12 is a nickel wire or a copper wire; the ceramic core rod 1 is made of alumina having a purity of 75%-99%; and the ceramic core rod 1 has a solid structure or a partially hollow structure with blind holes at one end.

The invention is specifically implemented as follows:
(1) a ceramic green substrate, such as an alumina green substrate, is punched into a rectangle, four through holes are punched out at one end of a square sheet, a heating circuit 2 and a temperature measuring circuit 3 are printed on one surface of the ceramic green substrate, electrodes are printed on the other surface thereof, and the through holes are located in the electrodes and tightly filled with a slurry;
(2) adhesive glue is applied to one surface of the ceramic green substrate which is printed with the circuits, and the substrate is curlily coated onto the ceramic core rod 1 and then pressed by an isostatic pressing machine;
(3) the above fully pressed ceramic core rod 1 and ceramic green substrate are sintered and molded in a tunnel furnace at 1500-1800°C;
(4) the above sintered and molded ceramic heating rod is immersed in a protective coating liquid, oven-dried, and then sintered in a tunnel furnace at 600-1700°C;
(5) the above fully sintered ceramic heating rod has a layer of metallic nickel deposited on bonding pads 5 by means of electroplating or chemical plating; and
(6) the above ceramic heating rod with nickel deposited on the electrodes is equipped with a ceramic flange 8 which is fixedly bonded by a high temperature resistant adhesive, nickel or copper leads are mounted on the electrodes, each of the bonding pads 5 is equipped with one metal lead 12, silver or a silver-copper solder is applied onto the electrodes and the leads near the electrodes, and the leads are brazed in a tunnel furnace at 600-1200°C.

The above technical solution merely represent a preferred technical solution of the invention, and some possible changes made to certain parts therein by those skilled in the art embody the principles of the invention and belong to the protection scope of the invention.

## Claims

1. A round rod-shaped ceramic heating element for an electronic cigarette, comprising a ceramic core rod (1), wherein a No. 1 ceramic substrate (4) is arranged at an outermost side of the ceramic core rod (1), a heating circuit (2) is arranged at an inner side of the No. 1 ceramic substrate (4), a No. 2 ceramic substrate (11) is arranged at an inner side of the heating circuit (2), a temperature measuring circuit (3) is arranged between the No. 2 ceramic substrate (11) and the ceramic core rod (1), a plurality of bonding pads (5) are arranged at an upper end of an outer circumferential surface of the No. 1 substrate (4), leads (7) are arranged above the bonding pads (5), a ceramic flange (8) is sleeved on an outer circumferential surface of the ceramic core rod (1), and each of the bonding pads (5) is provided with a metal lead (12).

2. The round rod-shaped ceramic heating element for an electronic cigarette according to claim 1, wherein a heating resistor (10) is arranged at an outer side of the ceramic core rod (1) and the inner side of the No. 1 substrate (4), and a temperature measuring resistor (9) is arranged beside the heating resistor (10).

3. The round rod-shaped ceramic heating element for an electronic cigarette according to claim 1, wherein the ceramic core rod (1) has a round rod-shaped tip structure with a tip angle of 30-140°.

4. The round rod-shaped ceramic heating element for an electronic cigarette according to claim 1, wherein the metal lead (12) is a nickel wire or a copper wire.

5. The round rod-shaped ceramic heating element for an electronic cigarette according to claim 1, wherein the ceramic core rod (1) has a solid structure or a partially hollow structure with blind holes at one end.

6. The round rod-shaped ceramic heating element for an electronic cigarette according to claim 1, wherein the ceramic core rod (1) is a ceramic core rod made of alumina having a purity of 75%-99%.

7. The round rod-shaped ceramic heating element for an electronic cigarette according to claim 1, wherein a protective coating (6) is arranged on the outer circumferential surface of the ceramic core rod (1), and the protective coating (6) is an oleophobic glass glaze.
